(19) Europäisches Patentamt / European Patent Office / Office européen des brevets

(11) **EP 3 949 832 A1**

(12) **EUROPEAN PATENT APPLICATION**
published in accordance with Art. 153(4) EPC

(43) Date of publication:
**09.02.2022 Bulletin 2022/06**

(21) Application number: **20783253.6**

(22) Date of filing: **05.03.2020**

(51) International Patent Classification (IPC):
**A61B 5/00** (2006.01)  **A61B 5/11** (2006.01)
**G16H 50/20** (2018.01)

(52) Cooperative Patent Classification (CPC):
**A61B 5/00; A61B 5/11; G16H 50/20**

(86) International application number:
**PCT/JP2020/009424**

(87) International publication number:
**WO 2020/203015 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2019 JP 2019070524**

(71) Applicant: **Cross Sync, Inc.**
**Yokohama-shi, Kanagawa 236-0004 (JP)**

(72) Inventor: **TAKAKI, Shunsuke**
**Yokohama-shi, Kanagawa 236-0004 (JP)**

(74) Representative: **TBK**
**Bavariaring 4-6**
**80336 München (DE)**

(54) **ILLNESS AGGRAVATION ESTIMATION SYSTEM**

(57) There is provided an aggravation estimation system for accurately estimating aggravation of a patient. The aggravation estimation system acquires bed image data chronologically capturing a capturing region including a bed of a patient, analyzes movement of the patient or a body part of the patient captured in the acquired bed image data, and estimates aggravation of the patient by performing, based on the movement that is determined by analysis, scoring in relation to at least one of oxygen administration or a state of consciousness included in indices of national early warning score.

FIG.3

|  | 3 | 2 | 1 | 0 | 1 | 2 | 3 |
|---|---|---|---|---|---|---|---|
| RESPIRATORY RATE | $\leqq$8 |  | 9—11 | 12—20 |  | 21—24 | $\geqq$25 |
| DEGREE OF OXYGEN SATURATION | $\leqq$91 | 92—93 | 94—95 | $\geqq$96 |  |  |  |
| OXYGEN ADMINISTRATION |  | Yes |  | No |  |  |  |
| BODY TEMPERATURE | $\leqq$35.0 |  | 35.1—36.0 | 36.1—38.0 | 38.1—39.0 | $\geqq$39.1 |  |
| SYSTOLIC BLOOD PRESSURE | $\leqq$90 | 91—100 | 101—110 | 111—219 |  |  | $\geqq$220 |
| HEART RATE | $\leqq$40 |  | 41—50 | 51—90 | 91—110 | 111—130 | $\geqq$131 |
| STATE OF CONSCIOUSNESS |  |  |  | A |  |  | V, P, or U |

EP 3 949 832 A1

**Description**

TECHNICAL FIELD

The present invention relates to an aggravation estimation system.

BACKGROUND ART

Recently, in the medical field, there has been research and development carried out for utilizing biological information of a certain patient measured therefrom not only for treatment, diagnosis, and the like of the patient but also for treatment, diagnosis, and the like of other patients as well.

As such kind of technology, the inventor of the present invention has already invented and filed a patent application for a device for accurately estimating aggravation of a patient based on various pieces of information regarding the patient (see Patent Document 1 below).

The invention according to Patent Document 1 is for generating a two-dimensional model that takes two variables (a first variable and a second variable) based on patient information as the vertical axis and the horizontal axis, respectively, deriving the first variable and the second variable from the patient information that is successively acquired to draw plots on a static scoring model, and estimating a point in time at which the plots are drawn in a specific region of the static scoring model as a timing at which the patient is aggravated.

CITATION LIST

PATENT DOCUMENTS

Patent Document 1: Japanese Patent Laid-Open No. 2019-17998

SUMMARY OF THE INVENTION

PROBLEM TO BE SOLVED BY THE INVENTION

Patent Document 1 discloses a mode in which the invention according to Patent Document 1 is implemented by performing scoring based on modified early warning scores (MEWS) that are derived using five items in the patient information, namely, a heart rate, a systolic blood pressure, a respiratory rate, a degree of oxygen saturation and a body temperature.

By contrast, national early warning score (NEWS) that is a British standard is widely known, and the national early warning score takes into account two items of oxygen administration and a state of consciousness, in addition to the five items mentioned above.

The invention according to Patent Document 1 leaves room for improvement in that the two items of oxygen administration and the state of consciousness are not sufficiently taken into account for estimation of aggravation of a patient.

The present invention, which is designed in view of the foregoing problem, provides an aggravation estimation system for accurately estimating aggravation of a patient.

MEANS FOR SOLVING THE PROBLEM

According to the present invention, there is provided an aggravation estimation system for estimating aggravation of a patient by performing scoring for a plurality of types of indices based on patient information chronologically acquired for the patient, the aggravation estimation system including an image acquisition unit that acquires, as the patient information, bed image data that is obtained by chronologically capturing a capturing region including a bed of the patient; an image analysis unit that takes, as a first analysis target, the patient or a body part of the patient captured in the bed image data that is acquired, and that analyzes movement of the first analysis target by an image analysis process on a plurality of pieces of the bed image data; and an aggravation estimation unit that estimates aggravation of the patient by performing, based on the movement of the first analysis target that is determined by analysis, scoring in relation to at least one of oxygen administration or a state of consciousness included in the plurality of types of indices.

According to the present invention, scoring of oxygen administration or the state of consciousness, both of which being indices of the national early warning score that are not sufficiently taken into account by the related invention, may be performed by computer processing, and aggravation of a patient may be estimated based on the score. Accordingly, the present invention may increase the accuracy of estimation of aggravation that uses the national early warning score, and may also reduce a working cost of healthcare personnel that occurs at the time of estimation of aggravation.

EFFECT OF THE INVENTION

The present invention provides an aggravation estimation system for accurately estimating aggravation of a patient.

BRIEF DESCRIPTION OF THE DRAWINGS

Fig. 1 is a configuration diagram of an aggravation estimation system.
Fig. 2 is a diagram schematically showing an installation state of a camera.
Fig. 3 is a conceptual diagram related to scoring of national early warning score.
Fig. 4 is a chart showing a specific example of a display screen of a computer terminal.
Fig. 5A is a diagram showing the number of pixels determined to be a foreground region by background subtraction, and Fig. 5B is a diagram specifying parts where the number of pixels changed in such a way that movement of an arm of a patient or of a nurse can be determined.
Figs. 6A and 6B are diagrams showing actual bed image data in a time zone surrounded by a long dashed short dashed line.
Figs. 7A to 7C are diagrams showing actual bed image data in a time zone surrounded by a long dashed short dashed line.

DESCRIPTION OF EMBODIMENTS

Hereinafter, an embodiment of the present invention will be described by referring to the accompanying drawings. Note that the same reference signs are applied to the same structural elements all through the drawings, and explanations thereof will not be repeated.

<Aggravation Estimation System 100 according to Present Invention>

First, an aggravation estimation system 100 according to the present invention will be described.

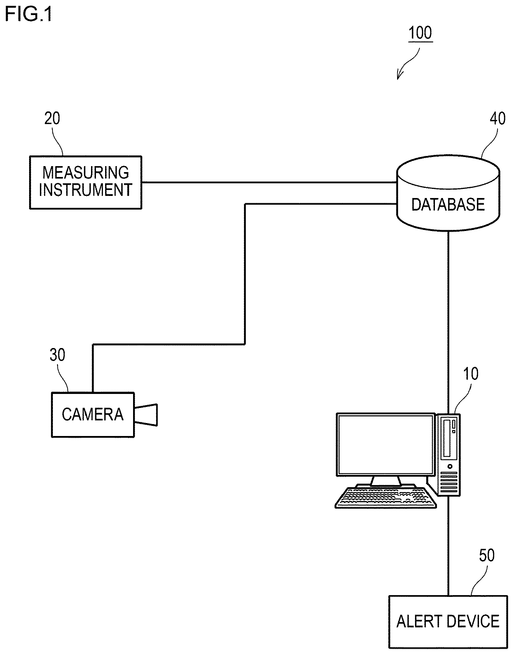

Fig. 1 is a configuration diagram of the aggravation estimation system 100.
The aggravation estimation system 100 is capable of estimating aggravation of a patient by performing scoring for a plurality of types of indices based on patient information that is chronologically acquired for the patient.
As shown in Fig. 1, the aggravation estimation system 100 includes a computer terminal 10, a measuring instrument 20, a camera 30, a database 40, and an alert device 50.

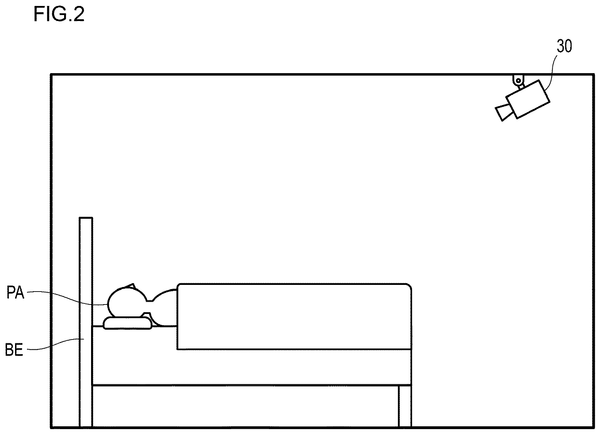

Fig. 2 is a diagram schematically showing an installation state of the camera 30.
As shown in Fig. 2, the camera 30 is installed such that at least a bed BE of a patient PA is included in a capturing range (a field of view), and the camera 30 chronologically captures a capturing region including the bed BE. Additionally, Figs. 1 and 2 show one camera 30, but the number of cameras 30 installed for one bed BE may be more than one, and the number is not particularly specified.
Image data that is captured by the camera 30 and that includes the bed BE in the capturing range (hereinafter referred to as "bed image data") is stored in the database 40.
The measuring instrument 20 chronologically measures biological information (vital signs) of the patient PA. The biological information of the patient PA here corresponds to a body temperature, a heart rate, a respiratory rate, a degree of oxygen saturation, a blood pressure (may be any of a systolic blood pressure, a diastolic blood pressure, and a mean blood pressure) and the like. Additionally, Fig. 1 shows one measuring instrument 20, but the number of measuring instruments 20 installed for one patient PA may be more than one, and the number is not particularly specified.
The biological information that is measured by the measuring instrument 20 is stored in the database 40.
The database 40 accumulates patient information that is chronologically acquired for the patient PA. The database 40 may be a dedicated database that is provided to introduce the present invention or may be a general database provided for a system (for example, an electronic medical record system) that is introduced separately from the present invention.
The patient information accumulated in the database 40 includes, in addition to the bed image data and the biological information described above, information regarding personal attributes of the patient (for example, name of the patient, sex, age, name of disease, identification number of the patient, and the like).
The computer terminal 10 is a device that estimates aggravation of the patient PA, and is capable of performing the following processes.

First, the computer terminal 10 derives a variable regarding a shock index and a variable regarding the national early warning score (NEWS) based on past patient information of the patient PA accumulated in the database 40, and generates a two-dimensional model that takes one of the variables as the horizontal axis and the other of the variables as the vertical axis.

Next, the computer terminal 10 acquires the patient information of the patient PA per unit time, and successively draws plots on the two-dimensional model based on the acquired patient information.

Furthermore, the computer terminal 10 estimates at least part of points in time at which the plots are drawn in a specific region (such as a terminal zone TZ described later) set in the two-dimensional model as a timing at which the patient PA is aggravated.

Note that the shock index mentioned above is determined by dividing the heart rate by the systolic blood pressure, and the national early warning score is determined by the following method.

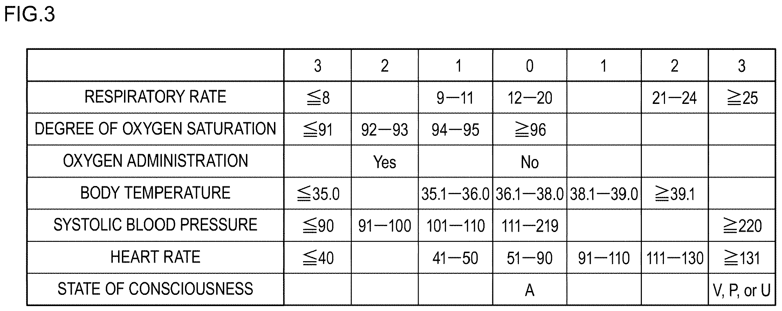

Fig. 3 is a conceptual diagram related to scoring of the national early warning score.

The national early warning score is determined by performing scoring of each of items including the respiratory rate, the degree of oxygen saturation, oxygen administration, the body temperature, the systolic blood pressure, the heart rate, and a state of consciousness per unit time, and by adding up the scores.

In relation to the respiratory rate, scores are 3 points for equal to or less than 8 times, 1 point for equal to or more than 9 times and equal to or less than 11 times, 0 points for equal to or more than 12 times and equal to or less than 20 times, 2 points for equal to or more than 21 times and equal to or less than 24 times, and 3 points for equal to or more than 25 times.

In relation to the degree of oxygen saturation, scores are 3 points for equal to or less than 91%, 2 points for equal to or more than 92% and equal to or less than 93%, 1 point for equal to or more than 94% and equal to or less than 95%, and 0 points for equal to or more than 96%.

In relation to oxygen administration, scores are 2 points if oxygen is administered, and 0 points if oxygen is not administered.

In relation to the body temperature, scores are 3 points for equal to or less than 35.0 degrees Celsius, 1 point for equal to or more than 35.1 degrees Celsius and equal to or less than 36.0 degrees Celsius, 0 points for equal to or more than 36.1 degrees Celsius and equal to or less than 38.0 degrees Celsius, 1 point for equal to or more than 38.1 degrees Celsius and equal to or less than 39.0 degrees Celsius, and 2 points for equal to or more than 39.1 degrees Celsius.

In relation to the systolic blood pressure, scores are 3 points for equal to or less than 90, 2 points for equal to or more than 91 and equal to or less than 100, 1 point for equal to or more than 101 and equal to or less than 110, 0 points for equal to or more than 111 and equal to or less than 219, and 3 points for equal to or more than 220.

In relation to the heart rate, scores are 3 points for equal to or less than 40 times, 1 point for equal to or more than 41 times and equal to or less than 50 times, 0 points for equal to or more than 51 times and equal to or less than 90 times, 1 point for equal to or more than 91 times and equal to or less than 110 times, 2 points for equal to or more than 111 times and equal to or less than 130 times, and 3 points for equal to or more than 131 times.

In relation to the state of consciousness, scores are 0 points if alert, and 3 points for other states (voice, pain, unresponsive).

Due to such manner of derivation, the national early warning score is determined as an integer of 0 (zero) or larger.

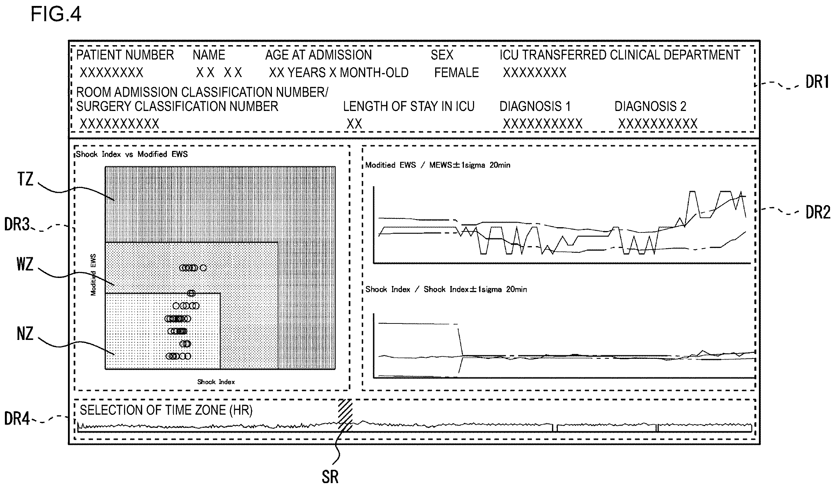

Fig. 4 is a chart showing a specific example of a display screen of the computer terminal 10.

As shown in Fig. 4, the display screen of the computer terminal 10 may be divided mainly into four parts. In Fig. 4, these four parts are shown by being surrounded by broken lines. Note that the broken lines are not actually displayed.

A patient attribute display part DR1 located in the uppermost part is a region that displays information related to personal attributes of the patient PA. In the present embodiment, displayed in the patient attribute display part DR1 are the identification number of the patient, name, age at the time of admission, sex, transferred clinical department, room admission classification number or surgery classification number, length of stay, and names of diagnosed diseases.

Additionally, names of a plurality of diagnosed diseases may be displayed, and Fig. 4 shows a state where two types are displayed, but the number may be changed as appropriate.

An index transition display part DR2 located on the right side of the middle part is a region that displays chronological changes in the national early warning score and the shock index. In the display of the index transition display part DR2 of the present embodiment, the vertical axis is the national early warning score or the shock index, and the horizontal axis is the time. Transitions of the national early warning score and the shock index are shown with solid lines, transitions of values in which $+1\sigma$ is added to each thereof are shown with long dashed short dashed lines, and transitions of values in which $-1\sigma$ is added to each thereof are shown with long dashed double-short dashed lines. Note here that "$\sigma$" is a standard deviation of the national early warning score or the shock index.

By observing chronological changes in the national early warning score and the shock index described above, it is possible to predict whether the condition of the patient will get worse or better than the current condition. Furthermore, through analyzing variations in the national early warning score and the shock index by referring also to the values with $\pm1\sigma$ added thereto, it is possible to predict the condition of the patient with still higher accuracy.

A model display part DR3 located on the left side of the middle part is a region that displays the two-dimensional model described above. The two-dimensional model according to the present embodiment is sectioned into three regions that are a stable condition zone NZ, a warning zone WZ, and a terminal zone TZ. The stable condition zone NZ is a region showing that the condition of the patient PA is most stable, and the terminal zone TZ is a region showing that the condition of the patient PA is most critical. The computer terminal 10 estimates the plotting time to the terminal zone TZ as the timing at which the subject is aggravated. That is, the terminal zone TZ corresponds to the specific region described above.

Even when a plot is drawn in the terminal zone TZ, aggravation does not have to be immediately estimated. This is because the possibility of aggravation is considered to be low when the condition is recovered immediately even if the condition of the patient PA temporarily changes and a plot is drawn in the terminal zone TZ. Therefore, aggravation of the patient PA may be estimated when plots are drawn in the terminal zone TZ over a specific length of time (when a specific number of plots are continuously drawn in the terminal zone TZ).

Additionally, the three regions as described above are shown in the drawing to be distinguishable in the present embodiment for the sake of explanations, but computer terminal 10 does not necessarily need to display each of the regions to be distinguishable.

A time zone display part DR4 located in the lowermost part is a region that displays which part among the whole time the time zone being displayed at that point in time in the index transition display part DR2 corresponds to. More specifically, a selected region SR (hatched part in the time zone display part DR4) out of the whole time corresponds to the time zone displayed in the index transition display part DR2.

Various types of information displayed on the display screen of the computer terminal 10 as described above are generated based on the patient information of the patient PA accumulated in the database 40. Thereby, "visualization" of the state (condition) of the whole body of the patient PA may be implemented.

The alert device 50 outputs an alert to inform a healthcare personnel of aggravation of the patient PA in the case where aggravation of the patient PA is estimated by the computer terminal 10, or in other words, in the case where one of the national early warning score and the shock index exceeds a threshold that is set as a lower limit of the terminal zone TZ.

Of the indices of the national early warning score used to estimate aggravation of the patient PA described above, the respiratory rate, the degree of oxygen saturation, the body temperature, the systolic blood pressure, and the heart rate are indices that can be measured by the measuring instrument 20, and thus, the computer terminal 10 may perform scoring using measurement values from the measuring instrument 20.

However, oxygen administration and the state of consciousness of the national early warning score are indices that cannot be measured by the measuring instrument 20, and thus, the computer terminal 10 performs the following processes.

<Scoring of Oxygen Administration>

To perform scoring of oxygen administration, the computer terminal 10 performs an image analysis process on the bed image data of the patient PA accumulated in the database 40, and determines whether oxygen is being administered to the patient PA (whether an oxygen mask worn by the patient PA is not removed).

Specifically, movement of the patient PA is detected by a method called background subtraction, and movement of an arm of the patient PA toward a facial range is detected. The background subtraction here is a method of comparing an original image B as a background and a target image I including a background region with a detection target object (in this case, the arm of the patient PA) as a foreground region, to thereby extract the detection target object that is not included in the background.

The background subtraction is performed by comparing a pixel value B(p) of the original image B and a pixel value I(p) of the target image I. Note that, in the case of background subtraction that uses grayscale-converted images, a brightness value is taken as the pixel value.

A pixel value includes noise even in a case of a still background, and thus, in most cases, the pixel value B(p) and the pixel value I(p) do not match completely even in the background region. Accordingly, the computer terminal 10 determines a region where a difference $\text{Diff}(B(p), I(p))$ between the pixel values is equal to or less than a threshold $\theta$ to be the background region, and determines a region where the difference $\text{Diff}(B(p), I(p))$ between the pixel values is equal to or greater than the threshold $\theta$ to be the foreground region.

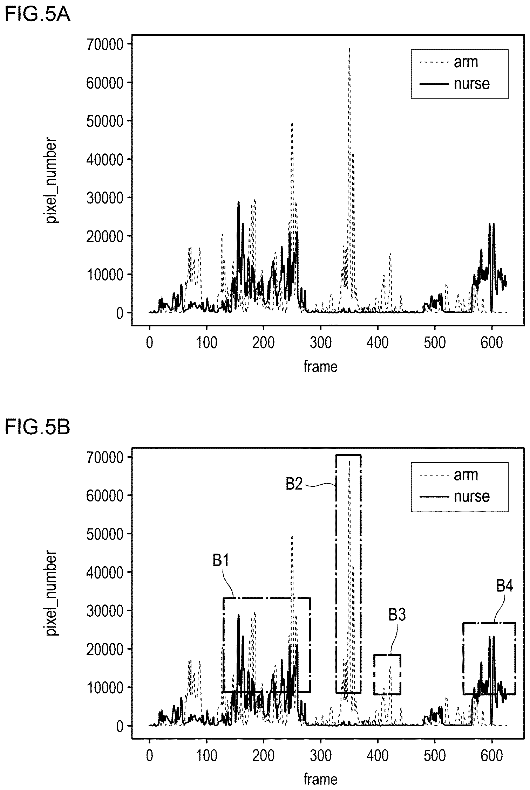

Fig. 5A is a diagram showing the number of pixels determined to be the foreground region by the background subtraction. Note that the number of pixels indicated by a broken line is the number of pixels in the foreground region that is recognized to be the arm of the patient PA, and the number of pixels indicated by a solid line is the number of pixels in the foreground region that is recognized to be a nurse NU.

The horizontal axis is a frame number of each bed image data that is the target of the image analysis process, or in other words, the frame number of target bed image data where the first bed image data is taken as a reference (the frame number = 0).

Fig. 5B is a diagram specifying parts, in Fig. 5A, where the number of pixels changed in such a way that movement of the arm of the patient PA or the nurse NU can be determined. Note that parts surrounded by long dashed short dashed lines B1 to B4 are the specified parts where the number of pixels changed.

In the part that is surrounded by the long dashed short dashed line B1, a change in the number of pixels is great for both the foreground region that is recognized to be the arm of the patient PA and the foreground region that is recognized to be the nurse NU.

That is, in the time zone when the bed image data that is the source of analysis of the corresponding part is captured, it is estimated that both the nurse NU and the arm of the patient PA are moving greatly.

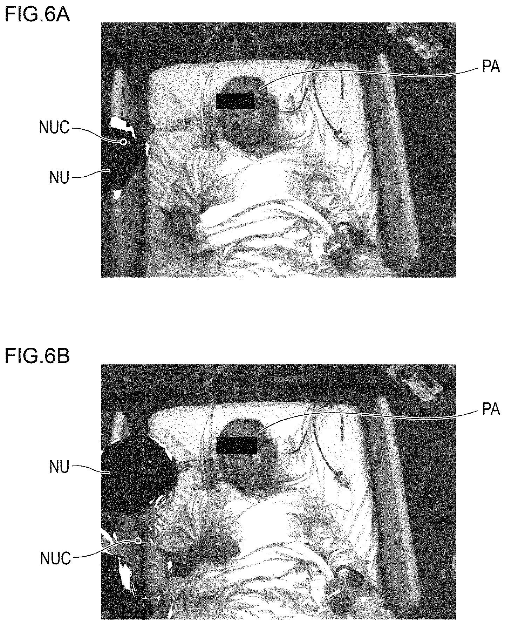

Figs. 6A and 6B are diagrams showing actual bed image data in a time zone surrounded by the long dashed short dashed line B1. Note that capturing is performed chronologically in the order of Fig. 6A and Fig. 6B.

Note that, in Figs. 6A and 6B, some of regions capturing the nurse NU are in white, and these are regions that are determined to be the foreground regions where the change in the number of pixels is great. Furthermore, white dots in Figs. 6A and 6B indicate centers of gravity of the white regions described above (hereinafter "center of gravity NUC").

Referring to Figs. 6A and 6B, it is clear that both the right arm of the patient PA and the nurse NU are moving (positions are chronologically moved) in the time zone surrounded by the long dashed short dashed line B1. When focusing particularly on the center of gravity NUC, it can be seen that the position of the center of gravity NUC is also moved.

That is, an estimation that both the nurse NU and the arm of the patient PA are moving in a time zone when the bed image data with a great change in the number of pixels is captured in relation to both the foreground region that is recognized to be the arm of the patient PA and the foreground region that is recognized to be the nurse NU can be said to be correct in this case.

The pieces of bed image data shown in Figs. 6A and 6B capture a state where the nurse NU is actually making patient rounds to the patient PA. As is clear from Figs. 6A and 6B, the patient PA is wearing an oxygen mask. Accordingly, even when a change in the number of pixels as that in the time zone surrounded by the long dashed short dashed line B1 is detected, the computer terminal 10 may determine 2 points, corresponding to "oxygen administered", as the score of oxygen administration.

In relation to the part surrounded by the long dashed short dashed line B2, there is no great change in the number of pixels for the foreground region that is recognized to be the nurse NU, but the change in the number of pixels is great for the foreground region that is recognized to be the arm of the patient PA.

That is, in the time zone when the bed image data that is the source of analysis of the corresponding part is captured, it is estimated that the arm of the patient PA is moving greatly, but that the nurse NU is not captured.

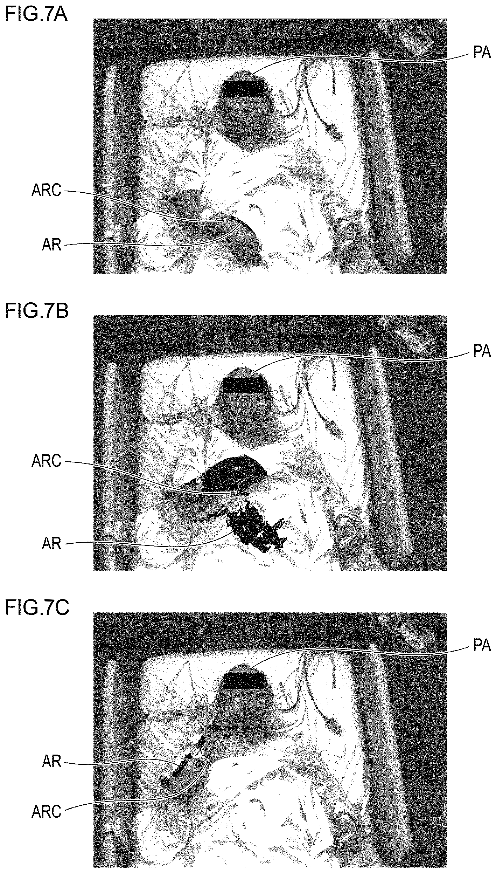

Figs. 7A to 7C are diagrams showing the actual bed image data in the time zone surrounded by the long dashed short dashed line B2. Note that capturing is performed chronologically in the order of Fig. 7A, Fig. 7B, and Fig. 7C.

Note that, in Figs. 7A to 7C, some of regions capturing the right arm of the patient PA are in black, and these are regions that are determined to be the foreground regions where the change in the number of pixels is great. Furthermore, black dots in Figs. 7A to 7C indicate centers of gravity of the black regions described above (hereinafter "center of gravity ARC").

Referring to Figs. 7A to 7C, it is clear that the right arm of the patient PA is moving (position is chronologically moved) in the time zone surrounded by the long dashed short dashed line B2. When focusing particularly on the center of gravity ARC, it can be seen that the position of the center of gravity ARC is also moved. Moreover, it is also clear that the nurse NU is not captured in this time zone.

That is, an estimation that the arm of the patient PA is moving in a time zone when the bed image data with a great change in the number of pixels is captured in relation to the foreground region that is recognized to be the arm of the patient PA can be said to be correct in this case. Furthermore, an estimation that the nurse NU is not captured in a time zone when the bed image data with a small change in the number of pixels is captured in relation to the foreground region that is recognized to be the nurse NU can be said to be correct in this case.

The pieces of bed image data shown in Figs. 7A to 7C actually capture an action of the patient PA touching the oxygen mask with the right hand. The oxygen mask is possibly removed by such an action.

Accordingly, in the case where a change in the number of pixels as that in the time zone surrounded by the long dashed short dashed line B2 is detected, the computer terminal 10 may determine 0 points, corresponding to "no oxygen administration", as the score of oxygen administration.

In the part surrounded by the long dashed short dashed line B3, there is no great change in the number of pixels for the foreground region that is recognized to be the nurse NU, but there is a change in the number of pixels for the foreground region that is recognized to be the arm of the patient PA. However, the change in the number of pixels in the part surrounded by the long dashed short dashed line B3 is smaller compared to the change in the number of pixels in the part surrounded by the long dashed short dashed line B2.

Accordingly, in the time zone when the bed image data that is the source of analysis of the corresponding part is captured, it is estimated that movement of the arm of the patient PA is relatively small, and that the nurse NU is not captured.

Although not shown in the drawings, the patient PA who is captured in the bed image data in this time zone keeps wearing the oxygen mask, and the nurse NU is not captured, and thus, it can be said that the estimation described above is correct.

Accordingly, in the case where a change in the number of pixels as that in the time zone surrounded by the long dashed short dashed line B3 is detected, the computer terminal 10 may determine 2 points, corresponding to "oxygen administered", as the score of oxygen administration.

In the part surrounded by the long dashed short dashed line B4, there is no great change in the number of pixels for the foreground region that is recognized to be the arm of the patient PA, but there is a change in the number of pixels for the foreground region that is recognized to be the nurse NU.

That is, in the time zone when the bed image data that is the source of analysis of the corresponding part is captured, it is estimated that the nurse NU is moving but movement of the arm of the patient PA is small.

Although not shown in the drawings, the patient PA who is captured in the bed image data in this time zone is sleeping with the oxygen mask on, and thus, movement of the patient PA is small, and the nurse NU is making patient rounds and is moving, and it can be said that the estimation described above is correct.

Accordingly, in the case where a change in the number of pixels as that in the time zone surrounded by the long dashed short dashed line B4 is detected, the computer terminal 10 may determine 2 points, corresponding to "oxygen administered", as the score of oxygen administration.

Additionally, the image analysis process performed for scoring of oxygen administration is illustrated as an example of the present embodiment, and may be changed as appropriate within a scope for achieving the object of the present invention (such as to a method of directly detecting presence/absence of the oxygen mask).

Moreover, the image analysis process may adopt a method used for machine learning (such as a convolutional neural network that takes, as teaching data, images showing presence/absence of an action of removing the oxygen mask after the patient moves his/her hand close to the face).

In the description of the image analysis process described above, a description is given of analysis of the movement of the arm of the patient PA that is performed by focusing on the change in the number of pixels in the foreground region extracted from the bed image data, but other elements may also be taken into account.

For example, when performing image analysis regarding movement of the arm of the patient PA, scoring of oxygen administration may be performed by extracting the face of the patient PA or the oxygen mask worn by the patient PA and by taking into account a distance between the extracted face of the patient PA or the extracted oxygen mask and the arm of the patient PA or whether a direction of movement of the arm of the patient PA is toward the face or the oxygen mask. The computer terminal 10 may thereby more accurately estimate administration/non-administration of oxygen (whether the oxygen mask is actually removed or not).

Additionally, assuming that image analysis can be performed in relation to both movement of the arm of the patient PA and whether the oxygen mask is worn or not, when the oxygen mask keeps being worn even when the arm of the patient PA is greatly moved, the computer terminal 10 may determine 2 points, corresponding to "oxygen administered", as the score of oxygen administration. Furthermore, in the case where a great movement of the arm of the patient PA and removal of the oxygen mask are extracted, the computer terminal 10 may determine 0 points, corresponding to "no oxygen administration", as the score of oxygen administration. That is, the score of oxygen administration is not changed merely by movement of the arm of the patient PA, and whether the oxygen mask is worn or not may be reflected in the score of oxygen administration.

In the description of the image analysis process given above, analysis of movement of the arm of the patient PA and movement of the nurse NU is performed in view of the risk of the oxygen mask being removed by the patient PA himself/herself, but analysis may alternatively be performed in view of other risks.

For example, movement of a third party who is a suspicious person (a person who is neither the patient nor a healthcare personnel) may be analyzed in view of the risk of the oxygen mask of the patient PA being removed by the third party.

<Scoring of State of Consciousness>

To perform scoring of the state of consciousness, the computer terminal 10 performs an image analysis process on the bed image data of the patient PA accumulated in the database 40, and determines whether the eyes of the patient PA are open or not.

Specifically, the computer terminal 10 first recognizes a facial region of the patient PA, recognizes regions corresponding to the eyes of the patient PA from the recognized facial region, and determines whether the eyes are open or closed.

In the case of determining that the eyes of the patient PA are open, the computer terminal 10 may determine 0 points, corresponding to "alert", as the score of the state of consciousness.

However, consciousness of the patient PA may be possibly clouded even when the eyes of the patient PA are open, and thus, the computer terminal 10 may further perform an image analysis process of extracting movement of pupils and irises from regions corresponding to the eyes of the patient PA, and determine the score of the state of consciousness by taking into account the extracted movement of the pupils and irises.

Note that, even when the eyes of the patient PA are determined to be closed, the patient PA may be merely sleeping, and the score of the state of consciousness cannot be immediately determined to be 3 points.

Accordingly, the computer terminal 10 may perform scoring of the state of consciousness by extracting movement of the patient PA or movement of a healthcare personnel in a region near the bed by the image analysis process using the background subtraction described above and by taking into account the extracted movement.

Additionally, the image analysis process performed for scoring of the state of consciousness described above is illustrated as an example of the present embodiment, and may be changed as appropriate within a scope for achieving the object of the present invention.

Moreover, the image analysis process may adopt a method used for machine learning (such as a convolutional neural network that takes, as teaching data, images capturing the patient).

A result of performing machine learning to generate a model for implementing the image analysis process for determining whether the eyes are open or closed is shown below, where 136 images obtained by capturing 17 patients are taken as teaching data.

[Table 1]

|  | F-measure | Recall | Precision |
|---|---|---|---|
| Eyes open | 0.620000 | 0.738095 | 0.534483 |
| Eyes closed | 0.773006 | 0.732558 | 0.818182 |

In the table shown above, Precision is a proportion of true data to data predicted to be true.

Recall is a proportion of data predicted to be true to data that are actually true.

F-measure is a harmonic mean of Precision and Recall, and may be calculated by the following equation.
[Math. 1]

$$F - measure = \frac{2 \cdot Recall \cdot Precision}{Recall + Precision} \qquad (1)$$

<Output of Alert by Alert Device 50>

As described above, the alert device 50 outputs an alert for informing a healthcare personnel of aggravation of the patient PA in a case where the national early warning score (a total value of scores of indices) exceeds a threshold that is set in advance (a value by which aggravation of the patient PA can be estimated).

That is, the alert device 50 basically outputs an alert when the national early warning score becomes high.

Now, assuming that estimation of aggravation is performed by the computer terminal 10, when focusing only on the change in the score of oxygen administration among indices according to the national early warning score, it cannot always be said that a high score (2 points) means a higher risk than a low score (0 points).

For example, in the case where there is an instruction from a doctor to administer oxygen to the patient PA, if removal of the oxygen mask by the patient PA wearing the oxygen mask is determined by analysis by the image analysis process, the score of oxygen administration is reduced from 2 points to 0 points although such a situation is not desirable.

With the national early warning score, scoring is performed not only for oxygen administration but also for the degree of oxygen saturation, and thus, even when the patient PA removes the oxygen mask that he/she is wearing, consistency is guaranteed by reflecting a change in the degree of oxygen saturation in the score. Accordingly, validity of output of an alert based on the national early warning score is not impaired.

However, in a case where it is analyzed and determined that there is occurrence of a situation that cannot always be said to be desirable, it is effective to output an alert by the alert device 50 for the purpose of urging a healthcare personnel to attach the oxygen mask on the patient PA.

In relation to such a purpose, in the case where a healthcare personnel (such as the nurse NU) is captured in the bed image data used for the image analysis process, there is no need to output the alert, and thus, the alert device 50 does not have to output the alert.

Additionally, the mode of alert to be output by the alert device 50 is not particularly specified, but because an alert based on the national early warning score and an alert based on removal of the oxygen mask are different in meaning, output is desirably performed in modes that can be distinguished from each other.

For example, an alert based on the national early warning score may be output to be recognizable in a relatively large region (for example, by being issued to a mobile phone carried by a doctor in charge in addition to a nurse station), and an alert based on removal of the oxygen mask may be output to be recognizable in a relatively small region (for example, by being issued only to the nurse station).

Alternatively, the alert based on the national early warning score and the alert based on removal of the oxygen mask may be distinguished from each other based on the level of volume in the case where the alerts are output as sound or based on the difference in the manner of display in the case where the alerts are displayed.

<Overview of Present Invention>

The aggravation estimation system 100 according to the present invention is summed up in the following.

The computer terminal 10 acquires, as the patient information, from the database 40, the bed image data chronologically capturing the capturing region including the bed BE of the patient PA.

The computer terminal 10 takes, as a first analysis target, the patient PA or a body part of the patient PA (such as an arm of the patient PA) captured in the bed image data that is acquired, and analyzes movement of the first analysis target by an image analysis process on a plurality of pieces of bed image data.

Additionally, the computer terminal 10 is described above to use the analysis result regarding the first analysis target to perform scoring of both oxygen administration and the state of consciousness among indices of the national early warning score, but in the present embodiment, it suffices if the analysis result is used to perform scoring of at least one of the two.

In other words, the computer terminal 10 estimates aggravation of the patient PA by performing, based on the movement of the first analysis target that is determined by analysis, scoring in relation to at least one of oxygen administration or the state of consciousness included in a plurality of types of indices.

Accordingly, the computer terminal 10 can be said to implement an image acquisition unit, an image analysis unit, and an aggravation estimation unit according to the present invention.

The aggravation estimation system 100 according to the present invention includes a configuration as described above, and thus, scoring of oxygen administration or the state of consciousness, both of which being indices of the national early warning score that are not sufficiently taken into account by the related invention, may be performed by computer processing, and aggravation of the patient PA is estimated based on the score.

Accordingly, the aggravation estimation system 100 according to the present invention may increase the accuracy of estimation of aggravation that uses the national early warning score, and may also reduce a working cost of healthcare personnel that occurs at the time of estimation of aggravation.

Furthermore, the computer terminal 10 can be said to extract at least one of the face of the patient PA or an oxygen administration device (the oxygen mask) that is worn by the patient PA, based on the image analysis process on the plurality of pieces of the bed image data, to analyze the movement of the first analysis target relative to the face of the patient PA or the oxygen administration device that is extracted, and to perform scoring of oxygen administration based on an analysis result regarding the movement of the first analysis target relative to the face of the patient PA or the oxygen administration device.

Accordingly, scoring may be performed for oxygen administration in better accordance with an actual situation, and accuracy of estimation of aggravation of the patient PA by the computer terminal 10 may be increased.

The computer terminal 10 takes, as a second analysis target, an eye of the patient PA that is captured in the bed image data that is acquired, and analyzes an eye open/closed state (whether the eye is open or not) of the patient PA by analyzing the second analysis target by the image analysis process on a plurality of pieces of the bed image data.

The computer terminal 10 performs scoring of the state of consciousness based on the eye open/closed state of the patient PA that is determined by analysis.

The aggravation estimation system 100 according to the present invention includes a configuration as described above, and thus, scoring of the state of consciousness that is an index of the national early warning score may be performed by computer processing.

Accordingly, the aggravation estimation system 100 according to the present invention may increase the accuracy of estimation of aggravation by reflecting the score of the state of consciousness, and may also reduce a working cost of healthcare personnel that occurs at the time of scoring.

The alert device 50 is capable of outputting two types of alerts as follows.

(1) The alert device 50 outputs an alert in a case where a total value of scores of a plurality of types of indices included in the national early warning score becomes higher than a predetermined value.

(2) The alert device 50 outputs an alert in a case where the score of oxygen administration is changed from a first value (2 points) to a second value (0 points) that is lower than the first value based on movement of the first analysis target (for example, the patient PA) determined by analysis.

Accordingly, the alert device 50 may implement a first alert output unit and a second alert output unit according to the present invention.

Furthermore, the computer terminal 10 can be said to take, as a third analysis target (for example, the nurse NU), healthcare personnel or a body part of the healthcare personnel captured in the bed image data that is acquired, and to analyze movement of the third analysis target by the image analysis process on a plurality of pieces of the bed image data.

Furthermore, in a case where movement of the third analysis target is determined from the bed image data by analysis, the alert device 50 can be said to restrict output of an alert even in a case where the score of oxygen administration is changed from the first value (2 points) to the second value (0 points) based on the movement of the first analysis target (for example, the patient PA) that is determined from the bed image data by analysis.

Accordingly, even in a case where the patient PA for whom oxygen administration is instructed by a doctor removes the oxygen mask himself/herself, a healthcare personnel may be urged as appropriate to attach the oxygen mask on the patient PA.

<Modifications of Present Invention>

The present invention has been described above with reference to the embodiment, but the present invention is not limited to the embodiment described above, and various changes and modifications may be made so long as the object of the present invention is achieved.

In the embodiment described above, a single computer terminal 10 is described to implement the image acquisition unit, the image analysis unit, and the aggravation estimation unit, but the structures may be implemented by different devices.

In the embodiment described above, in relation to the targets of image analysis process by the computer terminal 10, the first analysis target according to the present invention is described to be a body part of the patient (an arm of the patient), and the third analysis target is described to be the nurse himself/herself, but the embodiment of the present invention is not limited thereto.

For example, the first analysis target may be the patient himself/herself, and the third analysis target may be a healthcare personnel other than the nurse or a body part of the healthcare personnel.

Furthermore, in relation to the targets of image analysis process by the computer terminal 10, the second analysis target according to the present invention is described to be an eye of the patient, but this description does not deny analysis of the eye of the patient as the first analysis target, and the eye of the patient may be taken as the first analysis target in the embodiment of the present invention.

In the embodiment described above, a case is described where the oxygen administration device to be worn by the patient for oxygen administration is an oxygen mask, but instead, attachment/detachment of a nasal cannula to be inserted in nasal cavity or a mask with a reservoir bag (an oxygen mask with a storage bag) may be determined by image analysis process to thereby perform scoring of oxygen administration.

In this modification, the nasal cannula or the mask with a reservoir bag may be treated as a type of oxygen administration device.

Targets of analysis process by the computer terminal 10 cited in the embodiment described above are merely examples, and the analysis process may be performed taking other elements, not described, as the targets.

For example, another element that is captured in the bed image data (for example, a result of performing image analysis on text held by the healthcare personnel) may be used for the analysis process, or audio data that is recorded in synchronization with the bed image data may be used for the analysis process, or measurement data measured by the measuring instrument 20 or other pieces of data accumulated in the database 40 may be used for the analysis process.

In the embodiment described above, a mode is described where the device that estimates aggravation of a patient (the computer terminal 10) and the device that outputs an alert (the alert device 50) are different devices, but the devices may be integrated as one.

Furthermore, in the embodiment described above, a single device is described to output two patterns of alerts, but separate devices may output respective modes of alert.

The present embodiment includes the following technical ideas.

(1) An aggravation estimation system for estimating aggravation of a patient by performing scoring for a plurality of types of indices based on patient information chronologically acquired for the patient, the aggravation estimation system including: an image acquisition unit that acquires, as the patient information, bed image data that is obtained by chronologically capturing a capturing region including a bed of the patient; an image analysis unit that takes, as a first analysis target, the patient or a body part of the patient captured in the bed image data that is acquired, and that analyzes movement of the first analysis target by an image analysis process on a plurality of pieces of the bed image data; and an aggravation estimation unit that estimates aggravation of the patient by performing, based on the movement of the first analysis target that is determined by analysis, scoring in relation to at least one of oxygen administration or a state of consciousness included in the plurality of types of indices.

(2) The aggravation estimation system according to (1), where the image analysis unit extracts at least one of a face of the patient or an oxygen administration device that is worn by the patient, based on the image analysis process on a plurality of pieces of the bed image data, and analyzes the movement of the first analysis target relative to the face of the patient or the oxygen administration device that is extracted, and the aggravation estimation unit performs scoring of oxygen administration based on an analysis result regarding the movement of the first analysis target relative to the face of the patient or the oxygen administration device.

(3) The aggravation estimation system according to (1) or (2), where the image analysis unit takes, as a second analysis target, an eye of the patient among objects captured in the bed image data that is acquired, and analyzes an eye open/closed state of the patient by analyzing the second analysis target by the image analysis process on a plurality of pieces of the bed image data, and the aggravation estimation unit performs scoring of the state of consciousness based on an analysis result regarding the eye open/closed state of the patient that is determined by analysis.

(4) The aggravation estimation system according to any one of (1) to (3), including: a first alert output unit that outputs an alert in a case where a total value of scores of the plurality of types of indices becomes higher than a predetermined value; and a second alert output unit that outputs an alert in a case where the aggravation estimation unit changes, based on the movement of the first analysis target determined by analysis by the image analysis unit, a score of oxygen administration from a first value to a second value that is lower than the first value.

(5) The aggravation estimation system according to (4), where the image analysis unit takes, as a third analysis target, a healthcare personnel or a body part of the healthcare personnel captured in the bed image data that is acquired, and analyzes movement of the third analysis target by the image analysis process on a plurality of pieces of the bed image data, and in a case where movement of the third analysis target is determined from the bed image data by analysis, the second alert output unit restricts output of the alert even in a case where the score of oxygen administration is changed from the first value to the second value based on the movement of the first analysis target that is determined from the bed image data by analysis.

This application claims the benefit of priority to Japanese Patent Application No. 2019-70524, filed on April 2, 2019, which is hereby incorporated by reference in its entirety.

REFERENCE SIGNS LIST

| 100 | Aggravation estimation system |
|------|-------------------------------|
| 10 | Computer terminal |
| 20 | Measuring instrument |
| 30 | Camera |
| 40 | Database |
| 50 | Alert device |
| BE | Bed |
| NU | Nurse |
| PA | Patient |

**Claims**

1. An aggravation estimation system for estimating aggravation of a patient by performing scoring for a plurality of types of indices based on patient information chronologically acquired for the patient, the aggravation estimation system comprising:

an image acquisition unit that acquires, as the patient information, bed image data that is obtained by chronologically capturing a capturing region including a bed of the patient;
an image analysis unit that takes, as a first analysis target, the patient or a body part of the patient captured in the bed image data that is acquired, and that analyzes movement of the first analysis target by an image analysis process on a plurality of pieces of the bed image data; and

an aggravation estimation unit that estimates aggravation of the patient by performing, based on the movement of the first analysis target that is determined by analysis, scoring in relation to at least one of oxygen administration or a state of consciousness included in the plurality of types of indices.

2. The aggravation estimation system according to claim 1, wherein

the image analysis unit extracts at least one of a face of the patient or an oxygen administration device that is worn by the patient, based on the image analysis process on a plurality of pieces of the bed image data, and analyzes the movement of the first analysis target relative to the face of the patient or the oxygen administration device that is extracted, and

the aggravation estimation unit performs scoring of oxygen administration based on an analysis result regarding the movement of the first analysis target relative to the face of the patient or the oxygen administration device.

3. The aggravation estimation system according to claim 1 or 2, wherein

the image analysis unit takes, as a second analysis target, an eye of the patient among objects captured in the bed image data that is acquired, and analyzes an eye open/closed state of the patient by analyzing the second analysis target by the image analysis process on a plurality of pieces of the bed image data, and

the aggravation estimation unit performs scoring of the state of consciousness based on an analysis result regarding the eye open/closed state of the patient that is determined by analysis.

4. The aggravation estimation system according to any one of claims 1 to 3, comprising:

a first alert output unit that outputs an alert in a case where a total value of scores of the plurality of types of indices becomes higher than a predetermined value; and

a second alert output unit that outputs an alert in a case where the aggravation estimation unit changes, based on the movement of the first analysis target determined by analysis by the image analysis unit, a score of oxygen administration from a first value to a second value that is lower than the first value.

5. The aggravation estimation system according to claim 4, wherein

the image analysis unit takes, as a third analysis target, a healthcare personnel or a body part of the healthcare personnel captured in the bed image data that is acquired, and analyzes movement of the third analysis target by the image analysis process on a plurality of pieces of the bed image data, and

in a case where movement of the third analysis target is determined from the bed image data by analysis, the second alert output unit restricts output of the alert even in a case where the score of oxygen administration is changed from the first value to the second value based on the movement of the first analysis target that is determined from the bed image data by analysis.

FIG.1

FIG.2

FIG.3

| | 3 | 2 | 1 | 0 | 1 | 2 | 3 |
|---|---|---|---|---|---|---|---|
| RESPIRATORY RATE | ≦8 | | 9—11 | 12—20 | | 21—24 | ≧25 |
| DEGREE OF OXYGEN SATURATION | ≦91 | 92—93 | 94—95 | ≧96 | | | |
| OXYGEN ADMINISTRATION | | Yes | | No | | | |
| BODY TEMPERATURE | ≦35.0 | | 35.1—36.0 | 36.1—38.0 | 38.1—39.0 | ≧39.1 | |
| SYSTOLIC BLOOD PRESSURE | ≦90 | 91—100 | 101—110 | 111—219 | | | ≧220 |
| HEART RATE | ≦40 | | 41—50 | 51—90 | 91—110 | 111—130 | ≧131 |
| STATE OF CONSCIOUSNESS | | | | A | | | V, P, or U |

EP 3 949 832 A1

FIG.4

| | | | | | |
| PATIENT NUMBER | NAME | AGE AT ADMISSION | SEX | ICU TRANSFERRED | CLINICAL DEPARTMENT |
| XXXXXXXX | X X  X X | XX YEARS X MONTH-OLD | FEMALE | XXXXXXXX | |

ROOM ADMISSION CLASSIFICATION NUMBER/
SURGERY CLASSIFICATION NUMBER        LENGTH OF STAY IN ICU      DIAGNOSIS 1      DIAGNOSIS 2
XXXXXXXXXX                                          XX                     XXXXXXXXXX      XXXXXXXXXX

DR1

Shock Index vs Modified EWS

TZ
DR3
WZ
NZ

Modified EWS
Shock Index

Moditied EWS / MEWS±1sigma 20min

Shock Index / Shock Index±1sigma 20min

DR2

SELECTION OF TIME ZONE (HR)

DR4

SR

EP 3 949 832 A1

FIG.5A

FIG.5B

FIG.6A

FIG.6B

FIG.7A

FIG.7B

FIG.7C

**INTERNATIONAL SEARCH REPORT**

International application No.

PCT/JP2020/009424

A. CLASSIFICATION OF SUBJECT MATTER
A61B 5/00(2006.01)i; A61B 5/11(2006.01)i; G16H 50/20(2018.01)i
FI: A61B5/00 102A; A61B5/11 230; G16H50/20

According to International Patent Classification (IPC) or to both national classification and IPC

B. FIELDS SEARCHED

Minimum documentation searched (classification system followed by classification symbols)
A61B5/00-5/05, A61B5/06-5/22

Documentation searched other than minimum documentation to the extent that such documents are included in the fields searched
| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2019-502418 A (SPECTRAL MD, INC.) 31.01.2019 (2019-01-31) paragraphs [0001]-[0523], fig. 1-77 | 1-5 |
| A | JP 2012-509155 A (INSPIRE MEDICAL SYSTEMS, INC.) 19.04.2012 (2012-04-19) paragraphs [0001]-[0141], fig. 1-10 | 1-5 |
| A | WO 2019/009377 A1 (OMRON CORP.) 10.01.2019 (2019-01-10) paragraphs [0001]-[0055], fig. 1-7 | 1-5 |
| A | WO 2019/013094 A1 (YOKOHAMA CITY UNIVERSITY) 17.01.2019 (2019-01-17) paragraphs [0001]-[0046], fig. 1-2 | 1-5 |
| A | JP 2019-023790 A (YOKOHAMA CITY UNIVERSITY) 14.02.2019 (2019-02-14) paragraphs [0001]-[0065], fig. 1-13 | 1-5 |

☐ Further documents are listed in the continuation of Box C.   ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 April 2020 (28.04.2020) | 19 May 2020 (19.05.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

| International application No. |
| --- |
| PCT/JP2020/009424 |

| Patent Documents referred in the Report | Publication Date | Patent Family | Publication Date |
| --- | --- | --- | --- |
| JP 2019-502418 A | 31 Jan. 2019 | US 2017/0079530 A1 paragraphs [0001]-[0599], fig. 1-77 WO 2016/069788 A1 CN 107205624 A KR 10-2018-0078272 A | |
| JP 2012-509155 A | 19 Apr. 2012 | US 2011/0264164 A1 paragraphs [0001]-[0161], fig. 1-10 WO 2010/059839 A2 | |
| WO 2019/009377 A1 | 10 Jan. 2019 | JP 2019-16120 A paragraphs [0001]-[0055], fig. 1-7 | |
| WO 2019/013094 A1 | 17 Jan. 2019 | JP 2019-17998 A paragraphs [0001]-[0045], fig. 1-2 | |
| JP 2019-023790 A | 14 Feb. 2019 | (Family: none) | |

Form PCT/ISA/210 (patent family annex) (January 2015)

**REFERENCES CITED IN THE DESCRIPTION**

**Patent documents cited in the description**

- JP 2019017998 A
- JP 2019070524 A